# EUROPEAN PATENT APPLICATION

(11) **EP 3 682 876 A1**
(43) Date of publication of application: **22.07.2020**
(21) Application number: 18855260.8
(22) Date of filing: 08.06.2018
(51) Int. Cl.: A61K 31/34, A61K 31/135, A61K 9/24, A61K 9/28

(54) **PHARMACEUTICAL COMPOSITION FOR TREATING ACUTE AND CHRONIC PAIN, CONTAINING POLMACOXIB AND TRAMADOL**

(30) Priority: 15.09.2017 KR 20170118918
(71) Applicant: Crystalgenomics, Inc., Seongnam-si, Gyeonggi-do 13488 (KR)
(72) Inventor: CHO, Jae Pyoung, Gunpo-si Gyeonggi-do 15820 (KR); PARK, Hyunjin, Seoul 06784 (KR); CHO, Joong Myung, Seoul 05553 (KR)
(74) Representative: Schön, Christoph
(86) International application number: PCT/KR2018/006504
(87) International publication number: WO 2019/054606

(57) **Abstract**

The present invention relates to a complex comprising polmacoxib and tramadol. The present invention relates to a pharmaceutical composition and a medicine or an analgesic, all of which contain two types of active ingredients of polmacoxib and tramadol, and, more specifically, to effects and uses of the active ingredients in a medicine or an analgesic for the treatment of moderate acute and chronic pain caused by inflammatory and multiple factors.

## Description

### BACKGROUND OF THE INVENTION

### 1. Field of the Invention

The present invention relates to a composition comprising polmacoxib and tramadol, and more particularly, to a pharmaceutical composition for treating acute or chronic pain comprising polmacoxib used as a non-steroidal anti-inflammatory agent and tramadol as an opioid-based analgesic agent which shows excellent stability and excellent effect at low content.

### 2. Description of the Related Art

Pain is defined an unpleasant sensory and emotional experience associated with actual or potential tissue damage, or described in terms of such damage. It may also refer to sense of pain and sensory impairment caused by stimulation of the area in contact with cerebral cortex and marginal systemic regions through a neuropath consisting of nociceptors and nerve fibers. It can be said a warning response that transmits an abnormality inside or outside the body as a defense means to protect the body. Since pain itself is not a disease, elimination of pain does not cure the disease that causes pain.

Pain is largely divided into perceptual pain caused by damage or inflammation of somatic or visceral tissues, and neuropathic pain occurring after nerve injury. Perceptual pain may include skin pain, visceral pain, somatic pain, perceptual neuralgia, nerve root referred pain, somatic referred pain, and the like, and neuropathic pain may include pain due to peripheral or central nervous system dysfunction. If the pain persists for a long time or if the irritation is too severe, it may interfere with daily life and cause anxiety and fear. Because of this, people with chronic pain often have depression, so these characteristics should be considered in treatment.

There are many drugs known to be useful in the treatment of pain. Among them, opioids are used as an analgesic. Morphine derivatives are therapeutic agents that relieve pain in humans, of which analgesic effects are attained through the action of morphine receptors, preferably µ-opioid receptors. One of the widely used morphine derivatives that show good effects on oral administration is tramadol. Tramadol is an analgesic that acts by enhancing the activation of opioid receptors and the concentration of monoamine synapses in neurons. Tramadol having the chemical name 2-(dimethylaminomethyl)-1-(3-methoxyphenyl) has the structure of the following formula 1.

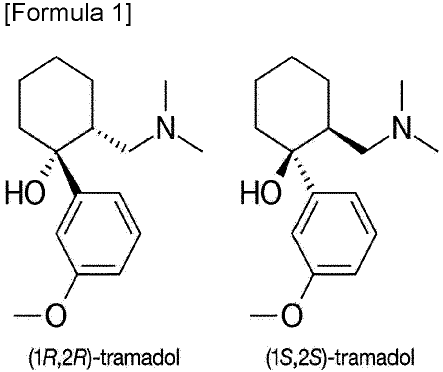

The pain-suppressing mechanism in which tramadol suppresses pain by attaching to a µ-receptor which causes pain, is similar to some narcotic analgesics. Accordingly, the prolonged use or injection of tramadol may lead to drug dependence. In addition, as a common symptom of side effects of tramadol, constipation, sleepless and anxiety symptom may occur. In addition, other side effects such as dizziness, drowsiness, nausea, etc. may occur. For this reason, the U.S. FDA has classified tramadol as a narcotic analgesic and recommends not to prescribe it for children under 12 years of age and lactating women in consideration of the risk of dyspnea. Because of this drawback, opioid drugs that have been used as an analgesic in the treatment of pain have not always been available in high doses or for repeated use.

As a result, in order to reduce the amount of opioids causing these side effects, it is often provided by combining opioids with drugs other than opioid analgesics. Among these combinations, interest in the drug composition in which a nonsteroidal anti-inflammatory agent (NSAID) and tramadol are combined has been reported (European Patent Publication No. 0546676).

U.S. patent No. 6,558,701 describes that for treating moderate to severe pain the International Health Organization has suggested a combination of opioid analgesics and nonsteroidal analgesics in order to exhibit effective pain relief and to reduce the amount of analgesics necessary for administration. As an alternative to increase the analgesic effect of the COX-2 selective inhibitor, Korean Patent No. 10-0444195 mentioned the synergistic effect of the coadministration of COX-2 selective inhibitors and opioids. According to the patent literature, with combination of COX-2 inhibitors and opioids, analgesic activity is increased and the dosage can be reduced compared to the case of individual use of both drugs for the same pain. Therefore, it was expected that the type and extent of side effects caused by each drug can be reduced.

One of the NSAIDs to be combined with tramadol is polmacoxib, which is currently commercially available under the name of ACELEX capsule, and has a structure represented by the following formula 2.

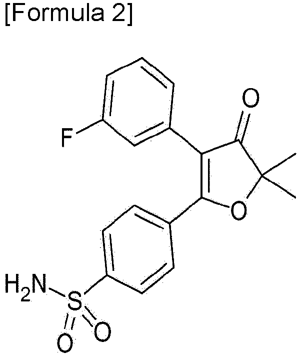

The compound name of polmacoxib, an active ingredient used in the pharmaceutical composition of the present invention, is 5-(4-(aminosulfonyl)-phenyl)-2,2-dimethyl-4-(3-fluorophenyl)-3(2H)-furanone. It is a selective inhibitor of COX-2 and has reduced gastrointestinal toxicities than conventional NSAIDs. It is known to be effective in inflammatory disease, inflammation-related disease, pain, solid cancer, angiogenesis-related disease, Alzheimer's disease, seizure and convulsion, stroke, or epilepsy (Korean Patent 10-0495389).

Currently, acetaminophen and celecoxib have been developed as a combination with tramadol. The above-mentioned polmacoxib exhibits has an advantage of maximum effects and minimum side effects even with a small amount of active ingredient.

COX (cyclooxygenase) is responsible for the production of prostaglandin. Two isoforms, COX-1 and COX-2, have been identified. COX-2 has been shown to be induced by pro-inflammatory stimuli, and is an isoform of an enzyme that is assumed to play a major role in the synthesis of prostanoid regulators of pain, inflammation, and fever.

The present invention intends to apply a combination of polmacoxib which is a COX-2 inhibitor and tramadol having these actions to the treatment of acute and chronic pain.

The focus of the application is on pharmaceutical compositions in which the combination of polmacoxib and tramadol can achieve additional effects in severe to moderate pain, in particular pain associated with inflammation.

### SUMMARY OF THE INVENTION

The inventors have noted synergistic effects on the pain and superior stabilities of a combination of polmacoxib and tramadol over existing products, and thus intended to formulate two different active ingredients into a single dosage form to further enhance the ease of taking each drug.

The present invention provides a pharmaceutical composition for treating acute and chronic pain, comprising polmacoxib and tramadol.

According to one embodiment, the pain may be neurological pain, including diabetic neuropathy, cancer pain, osteoarthritis, moderate pain, rheumatoid arthritis, spondylitis or sciatica.

According to one embodiment, the composition may be in the form of a double-layered tablet having a bi-layered structure in which the polmacoxib and the tramadol are separated into each layer.

According to one embodiment, the composition may further comprise a coating base.

According to one embodiment, the composition may be in the form of a cored tablet formulation containing an inner core of tramadol and an outer core of polmacoxib or a dual release micro-coating (DRM) containing an outer coating layer of polmacoxib on an inner layer of tramadol.

According to one embodiment, the ratio of polmacoxib to tramadol may be a weight ratio of 1: 1 to 500.

In addition, the ratio of polmacoxib to tramadol may be a weight ratio of 1: 1 to 300.

In addition, the ratio of polmacoxib to tramadol may be a weight ratio of 2: 1 to 300.

According to one embodiment, the composition may further comprise a pharmaceutically acceptable carrier.

Other specific embodiments of the present invention are included in the following detailed description.

### EFFECT OF THE INVENTION

In the present invention, polmacoxib and tramadol are formulated into a single dosage form containing rapid release of polmacoxib and sustained release of tramadol, which is a combination designed to allow the effects of each drug to be complementarily and sustainably exerted even with only one or two doses per day by minimizing the interaction of each drug in the dosage form.

Multi-layered tablet according to the present invention is suitable for mass production since it has excellent flow of particles, a hardness suitable for facilitating package, transport and handling of tablets, and less occurrence of capping or lamination of tablets during manufacturing process.

### BRIEF DESCRIPTION OF THE DRAWINGS

Figs. 1 to 12 are graphs showing the dissolution patterns for each Example.
Fig. 13 is a graph showing the weight change in the animal model.
Fig. 14 is a graph showing the pain index in the animal model.
Fig. 15 is a graph showing the anti-inflammatory factor activity in the animal model.
Figs. 16 and 17 show the histopathological autopsy results.

### DETAILED DESCRIPTION OF THE INVENTION

Since various modifications and variations can be made in the present invention, particular embodiments are illustrated in the drawings and will be described in detail in the detailed description. It should be understood, however, that the invention is not intended to be limited to the particular embodiments, but includes all modifications, equivalents, and alternatives falling within the spirit and scope of the invention. In the following description of the present invention, detailed description of known functions will be omitted if it is determined that it may obscure the gist of the present invention.

Hereinafter, the pharmaceutical composition according to the embodiment of the present invention will be described in more detail.

The term "pharmaceutical composition", as used herein may be described interchangeably with a "pharmacological composition" and a "pharmaceutically acceptable composition", and which means any inorganic or organic compound formulation which can be a relatively non-toxic to a subject to be administered and have harmless effective action. In addition, it may refer to any organic or inorganic compound formulation in that side effects resulting from the composition do not impair the efficacy of the drug, and that does not cause serious irritation to a subject to be administered by the compound and does not impair the biological activities and properties of the compound.

As used herein, the term 'subject to be administered' may be used interchangeably with 'individual to be administered' and 'organism to be administered', and may refer to any animal including humans in which acute or chronic pain is caused or may be caused.

The present invention provides a combination composition for treating acute and chronic pain, comprising polmacoxib and tramadol. The combination of polmacoxib and tramadol may have effects of reducing deformation of active ingredients to increase stabilities against changes over time when mixed with other pharmaceutically acceptable additives.

According to one embodiment, the ratio of polmacoxib to tramadol in the pharmaceutical composition according to the invention may be a weight ratio of 1 to 500: 1 to 500, for example a weight ratio of 1 to 300: 1 to 300, for example a weight ratio of 1 to 2: 1 to 300, for example a weight ratio of 1: 1 to 300, for example a weight ratio of 2: 1 to 300. Specifically, the content ratio of polmacoxib to tramadol may be 1: 500, and the mass ratio of polmacoxib to tramadol may be 1: 500, for example.

According to one embodiment, the composition may comprise 0.1 to 10% by weight of polmacoxib and 10 to 50% by weight of tramadol, for example 0.3 to 1% by weight of polmacoxib and 20 to 40% by weight of tramadol based on the total weight of the composition.

According to one embodiment, polmacoxib may be present in an amount of 1 to 5 mg, for example 1 to 2 mg, and tramadol may be present in an amount of 30 to 300 mg, for example 37.5 to 200 mg in the composition.

According to one embodiment, the present invention may further comprise a pharmaceutically acceptable and physiologically suitable additive. For example, any of the pharmaceutically acceptable additives commonly used in each formulation can be used as an additive, such as a filler, an extender, a binder, a disintegrant, a lubricant, a preservative, a buffer, a coating agent, a sweetener, a solubilizer, a suspending agent, a colorant, a water soluble additive, an excipient, a carrier, a filler, a glydent, a hygrocopic agent, etc. For example, the additive may be present in an amount of 5 to 90% by weight, for example 40 to 90% by weight based on the total weight of the composition.

According to one embodiment, the present invention can be divided into a polmacoxib compartment and a tramadol compartment. Specifically, the polmacoxib compartment may be composed of silicified microcrystalline cellulose, sodium starch glycolate, povidone, or magnesium stearate, but is not limited thereto. For example, since the polmacoxib compartment contains a small amount of the active ingredient, silicified microcrystalline cellulose may be used as a water-insoluble polymer in order to improve the mixing uniformity of the drug in the mixture and to improve the tableting ability, but the present invention is not limited thereto.

In addition, the tramadol layer for sustained release may be composed of microcrystalline cellulose, hydroxypropyl methylcellulose, povidone, silicon dioxide or magnesium stearate, but is not limited thereto. In general, as a means of delaying the release of the drug, there may be used cellulose derivatives, such as hydroxypropyl cellulose, methylcellulose, and hydroxypropyl methylcellulose and carboxymethylcellulose sodium or carboxyvinyl polymers, which are gel-forming substances that can control the release of the active ingredient by coating the drug with a water-insoluble substance or by contacting with water to form a hydrogel. For example, the tramadol layer may contain hydroxypropyl methylcellulose to implement sustained release, but is not limited thereto.

According to one embodiment, the present invention may further comprise a coating base to ensure long-term stability of the light-sensitive polmacoxib. Although commonly used coating bases can be used, for example, the coating bases may be at least one selected from the group consisting of a coating base including polyvinyl alcohol derivatives, methacrylic acid derivatives and polyacrylic acid derivatives, Opadry®, Kollicoat®, and hydroxypropyl methylcellulose (HPMC) among water-soluble coating bases, for example Opadry®. According to one embodiment, the composition according to the present invention may be provided in oral dosage form and may be formulated in solid or liquid form. In particular, the composition according to the invention may be provided in any convenient form, such as in the form of tablet, pellet, granule, capsule, suspension, emulsion or powder, which is suitable for reconstitution with water or other suitable liquid medium. For example, the composition of the present invention may be provided in the form of a tablet, such as a single layer and a multilayer tablet, or in the form of a dual release micro-coating (DRM).

According to one embodiment, the composition of the present invention may be a dual release micro-coating (DRM) containing an outer coating layer of polmacoxib on an inner layer of tramadol.

In addition, the composition of the present invention can be formulated in the form of cored tablets, double-layered tablets, multi-layer tablets, single tablets, capsules or suspensions. It is preferably physically separated into a polmacoxib compartment and a tramadol compartment so that the polmacoxib compartment shows rapid drug release and the tramadol compartment shows sustained drug release. For example, it may be formulated into a double-layered tablet of bi-layered structure having the separated polmacoxib layer and tramadol layer. The manufacturing method of the bi-layered structure may comprise, but is not limited to, forming a lower layer with tramadol and a mixture granule thereof and compressing it, and then forming an upper layer with polmacoxib and a mixture granule thereof and tableting it using a tableting machine.

In addition, it may be prepared in a cored tablet formulation containing an inner core of tramadol and an outer core of polmacoxib. Alternatively, the final formulation of the composition according to the invention may be selected from a cored tablet comprising an inner core composed of a sustained release compartment and an outer layer composed of a rapid release compartment, a capsule containing particles, granules, pellets or tablets composed of a sustained release compartment and particles, granules, pellets, or tablets composed of a rapid release compartment, or a multilayered tablet in which a layer of a sustained release compartment and a layer of a rapid release compartment are laminated.

According to one embodiment, the present invention may exhibit an effective effect as an analgesic for treating pain, in particular, acute pain and chronic pain. The pain may include neurological pain including diabetic neuropathy, hyperalgesia, allodynia, cancer pain and osteoarthritis as well as severe to moderate pain, rheumatoid arthritis, ankylosing spondylitis, sciatica and frozen shoulder. For example, the present invention can be used for the treatment of severe to moderate pain associated with inflammatory components such as rheumatoid arthritis, ankylosing spondylitis, sciatica and frozen shoulder.

The pharmacological or pharmaceutical composition according to the present invention may be prepared in any form suitable for application to humans, including infants, children and adult animals, by standard procedures known to those skilled in the art.

Hereinafter, embodiments of the present invention will be described in detail so that those skilled in the art can easily carry out the present invention. The present invention may, however, be embodied in many different forms and should not be construed as limited to the embodiments set forth herein.

### Example 1

Polmacoxib and tramadol as a main ingredient and excipients in Table 1 below were combined, dried, granulated, mixed and tableted into a wet granule form.

The polmacoxib layer proceeded by quoting a tablet prescription of the existing product of Crystal Genomics, and prescription study focused on the tramadol layer showing sustained release.

For initial sustained release of the tramadol layer of Example 1, HPMC K100M (METHOCEL ™ K100M Premium Hydroxypropyl methylcellulose, DOW Chemical Company) was used as a sustained release agent to prepare a bilayer tablet for oral administration.

**[Table 1]**

| Polmacoxib-tramadol combination (Example 1) | | | | | | |
|---|---|---|---|---|---|---|
| Tramadol layer | | Manufacturing process | Purpose | Material | per tablet (mg) | Content (%) |
| | 1 | First granulation | Main ingredient | Tramadol | 150 | 33.3 |
| | 2 | | Excipient | MCC 102 | 25 | 5.6 |
| | 3 | | Excipient | Lactose monohydrate | 30 | 6.7 |
| | 4 | | Sustained-release agent | HPMC K100M | 100 | 22.2 |
| | 5 | | Binder | PVP K-30 | 15 | 3.3 |
| | 6 | Post-mixing | Sustained-release agent | HPMC K100M | 100 | 22.2 |
| | 7 | | Disintegrant | L-HPC (NBD-020) | 25 | 5.6 |
| | 8 | | Lubricant | Magnesium stearate | 5 | 1.1 |
| | Total amount of uncoated tablet | | | | 450 | 100 |
| Polmacoxib layer | 9 | First granulation | Main ingredient | Polmacoxib | 1 | 2 |
| | 10 | | Disintegrant | Sodium starch glycolate | 3.5 | 7 |
| | 11 | | Excipient | Silicified | 43.5 | 87 |
| | | | | microcrystalline cellulose | | |
| | 12 | | Binder | Povidone K-30 | 1.5 | 3 |
| | 13 | Post-mixing | Lubricant | Magnesium stearate | 0.5 | 1 |
| | Total amount of uncoated tablet | | | | 50 | 100 |

### Example 2

In order to reduce the initial high release amount, in addition to HPMC K100M, Carbomer as a sustained-release agent were added in post-mixing to prepare a bilayer tablet for oral administration.

**[Table 2]**

| Polmacoxib-tramadol combination (Example 2) | | | | | | |
|---|---|---|---|---|---|---|
| Tramadol layer | | Manufacturing process | Purpose | Material | per tablet (mg) | Content (%) |
| | 1 | First granulation | Main ingredient | Tramadol | 150 | 28.6 |
| | 2 | | Excipient | MCC 102 | 25 | 4.8 |
| | 3 | | Excipient | Lactose monohydrate | 30 | 5.7 |
| | 4 | | Sustained-release agent | HPMC K100M | 200 | 38.1 |
| | 5 | | Binder | PVP K-30 | 15 | 2.9 |
| | 6 | Post-mixing | Sustained-release agent | HPMC K100M | 30 | 5.7 |
| | 7 | | Sustained-release agent | Carbo mer | 45 | 8.6 |
| | 8 | | Disintegrant | L-HPC | 25 | 4.8 |
| | 9 | | Lubricant | Magnesium stearate | 5 | 0.9 |
| | Total amount of uncoated tablet | | | | 525 | 100 |
| Polmacoxib layer | 10 | First granulation | Main ingredient | Polmacoxib | 1 | 2 |
| | 11 | | Disintegrant | Sodium starch glycolate | 3.5 | 7 |
| | 12 | | Excipient | Silicified microcrystalline cellulose | 43.5 | 87 |
| | 13 | | Binder | Povidone K-30 | 1.5 | 3 |
| | 14 | Post-mixing | Lubricant | Magnesium stearate | 0.5 | 1 |
| | Total amount of uncoated tablet | | | | 50 | 100 |

### Example 3

Ethylcellulose was added in post-mixing to prepare a bilayer tablet for oral administration.

**[Table 3]**

| Polmacoxib-tramadol combination (Example 3) | | | | | | |
|---|---|---|---|---|---|---|
| Tramadol layer | | Manufacturing process | Purpose | Material | per tablet (mg) | Content (%) |
| | 1 | First granulation | Main ingredient | Tramadol | 150 | 30.3 |
| | 2 | | Excipient | MCC 102 | 25 | 5.1 |
| | 3 | | Excipient | Lactose monohydrate | 30 | 6.1 |
| | 4 | | Sustained-release agent | HPMC K100M | 200 | 40.4 |
| | 5 | | Binder | PVP K-30 | 15 | 3 |
| | 6 | Post-mixing | Excipient | Ethylcellulose | 45 | 9.1 |
| | 7 | | Disintegrant | L-HPC | 25 | 5.1 |
| | 8 | | Lubricant | Magnesium stearate | 5 | 1 |
| | Total amount of uncoated tablet | | | | 495 | 100 |
| Polmacoxib layer | 9 | First granulation | Main ingredient | Polmacoxib | 1 | 2 |
| | 10 | | Disintegrant | Sodium starch glycolate | 3.5 | 7 |
| | 11 | | Excipient | Silicified microcrystalline cellulose | 43.5 | 87 |
| | 12 | | Binder | Povidone K-30 | 1.5 | 3 |
| | 13 | Post-mixing | Lubricant | Magnesium stearate | 0.5 | 1 |
| | Total amount of uncoated tablet | | | | 50 | 100 |

### Example 4

The binding force between the excipients was increased by increasing the content of the binder in the granulation process instead of the sustained release agent in the post-mixing. The content of PVP K-30 as the binder was increased to prepare a bilayer tablet for oral administration.

**[Table 4]**

| Polmacoxib-tramadol combination (Example 4) | | | | | | |
|---|---|---|---|---|---|---|
| Tramadol layer | | Manufacturing process | Purpose | Material | per tablet (mg) | Content (%) |
| | 1 | First granulation | Main ingredient | Tramadol | 150 | 32.3 |
| | 2 | | Excipient | MCC 102 | 25 | 5.4 |
| | 3 | | Excipient | Lactose monohydrate | 30 | 6.5 |
| | 4 | | Sustained-release agent | HPMC K100M | 200 | 43 |
| | 5 | | Binder | PVP K-30 | 30 | 6.5 |
| | 6 | Post-mixing | Disintegrant | L-HPC | 25 | 5.4 |
| | 7 | | Lubricant | Magnesium stearate | 5 | 1.1 |
| | Total amount of uncoated tablet | | | | 465 | 100 |
| Polmacoxib layer | 8 | First granulation | Main ingredient | Polmacoxib | 1 | 2 |
| | 9 | | Disintegrant | Sodium starch glycolate | 3.5 | 7 |
| | 10 | | Excipient | Silicified microcrystalline cellulose | 43.5 | 87 |
| | 11 | | Binder | Povidone K-30 | 1.5 | 3 |
| | 12 | Post-mixing | Lubricant | Magnesium stearate | 0.5 | 1 |
| | Total amount of uncoated tablet | | | | 50 | 100 |

### Example 5

The target dose of the tramadol layer was reduced as shown in Table 5 below to prepare a bilayer tablet for oral administration.

**[Table 5]**

| Polmacoxib-tramadol combination (Example 5) | | | | | | |
|---|---|---|---|---|---|---|
| Tramadol layer | | Manufacturing process | Purpose | Material | per tablet (mg) | Content (%) |
| | 1 | First granulation | Main ingredient | Tramadol | 75 | 33.33 |
| | 2 | | Excipient | MCC 102 | 12.5 | 5.56 |
| | 3 | | Excipient | Lactose monohydrate | 15 | 6.67 |
| | 4 | | Sustained-release agent | HPMC K100M | 50 | 22.22 |
| | 5 | | Binder | PVP K-30 | 7.5 | 3.33 |
| | 6 | Post-mixing | Sustained-release agent | HPMC K100M | 50 | 22.22 |
| | 7 | | Disintegrant | L-HPC (NBD-020) | 12.5 | 5.56 |
| | 8 | | Lubricant | Magnesium stearate | 2.5 | 1.11 |
| | Total amount of uncoated tablet | | | | 225 | 100 |
| Polmacoxib layer | 9 | First granulation | Main ingredient | Polmacoxib | 1 | 2 |
| | 10 | | Disintegrant | Sodium starch glycolate | 3.5 | 7 |
| | 11 | | Excipient | Silicified microcrystalline cellulose | 43.5 | 87 |
| | 12 | | Binder | Povidone K-30 | 1.5 | 3 |
| | 13 | Post-mixing | Lubricant | Magnesium stearate | 0.5 | 1 |
| | Total amount of uncoated tablet | | | | 50 | 100 |

### Example 6

As shown in Table 6 below, low-molecular HPMC was used as a sustained-release agent to prepare a bilayer tablet for oral administration.

**[Table 6]**

| Polmacoxib-tramadol combination (Example 6) | | | | | | |
|---|---|---|---|---|---|---|
| Tramadol layer | | Manufacturing process | Purpose | Material | per tablet (mg) | Content (%) |
| | 1 | First granulation | Main ingredient | Tramadol | 75 | 37.5 |
| | 2 | | Excipient | HPMC K100M | 15 | 7.5 |
| | 3 | | Excipient | HPMC K15M | 9 | 4.5 |
| | 4 | | Sustained-release agent | HPMC K4M | 3 | 1.5 |
| | 5 | | Binder | Lactose | 31.5 | 15.8 |
| | 6 | | Solubilizer | Microcrystalline cellulose | 30 | 15 |
| | 7 | | Sustained-release agent | PVP K30 | 9 | 4.5 |
| | 8 | Post-mixing | Disintegrant | Talc | 4.5 | 2.3 |
| | 9 | | Lubricant | Magnesium stearate | 3 | 1.5 |
| | Total amount of uncoated tablet | | | | 200 | 100 |
| Polmacoxib layer | 10 | First granulation | Main ingredient | Polmacoxib | 1 | 2 |
| | 11 | | Disintegrant | Sodium starch glycolate | 3.5 | 7 |
| | 12 | | Excipient | Silicified microcrystalline cellulose | 43.5 | 87 |
| | 13 | | Binder | Povidone K-30 | 1.5 | 3 |
| | 14 | Post-mixing | Lubricant | Magnesium stearate | 0.5 | 1 |
| | Total amount of uncoated tablet | | | | 50 | 100 |

### Example 7

Three kinds of HPMCs having different molecular weights were added in the amounts shown in Table 7 to prepare a bilayer tablet for oral administration.

**[Table 7]**

| Polmacoxib-tramadol combination (Example 7) | | | | | | |
|---|---|---|---|---|---|---|
| Tramadol layer | | Manufacturing process | Purpose | Material | per tablet (mg) | Content (%) |
| | 1 | First granulation | Main ingredient | Tramadol | 75 | 37.5 |
| | 2 | | Excipient | HPMC K100M | 15 | 7.5 |
| | 3 | | Excipient | HPMC K15M | 6 | 3 |
| | 4 | | Sustained-release agent | HPMC K4M | 6 | 3 |
| | 5 | | Binder | Lactose | 31.5 | 15.8 |
| | 6 | | Solubilizer | Microcrystalline cellulose | 30 | 15 |
| | 7 | | Sustained-release agent | PVP K30 | 9 | 4.5 |
| | 8 | Post-mixing | Disintegrant | Talc | 4.5 | 2.3 |
| | 9 | | Lubricant | Magnesium stearate | 3 | 1.5 |
| | Total amount of uncoated tablet | | | | 200 | 100 |
| Polmacoxib layer | 10 | First granulation | Main ingredient | Polmacoxib | 1 | 2 |
| | 11 | | Disintegrant | Sodium starch glycolate | 3.5 | 7 |
| | 12 | | Excipient | Silicified microcrystalline cellulose | 43.5 | 87 |
| | 13 | | Binder | Povidone K-30 | 1.5 | 3 |
| | 14 | Post-mixing | Lubricant | Magnesium stearate | 0.5 | 1 |
| | Total amount of uncoated tablet | | | | 50 | 100 |

### Example 8

The content ratio of HPMC K100M was increased to prepare a bilayer tablet for oral administration.

**[Table 8]**

| Polmacoxib-tramadol combination (Example 8) | | | | | | |
|---|---|---|---|---|---|---|
| Tramadol layer | | Manufacturing process | Purpose | Material | per tablet (mg) | Content (%) |
| | 1 | First granulation | Main ingredient | Tramadol | 75 | 37.5 |
| | 2 | | Excipient | MCC 102 | 15 | 7.5 |
| | 3 | | Sustained-release agent | HPMC K100M | 100 | 50 |
| | 4 | | Binder | PVP K-30 | 7.5 | 3.8 |
| | 5 | Post-mixing | Lubricant | Magnesium stearate | 2.5 | 1.3 |
| | Total amount of uncoated tablet | | | | 200 | 100 |
| Polmacoxib layer | 6 | First granulation | Main ingredient | Polmacoxib | 1 | 2 |
| | 7 | | Disintegrant | Sodium starch glycolate | 3.5 | 7 |
| | 8 | | Excipient | Silicified microcrystalline cellulose | 43.5 | 87 |
| | 9 | | Binder | Povidone K-30 | 1.5 | 3 |
| | 10 | Post-mixing | Lubricant | Magnesium stearate | 0.5 | 1 |
| | Total amount of uncoated tablet | | | | 50 | 100 |

### Example 9

Another sustained-release agent PEO (POLYOXtm WSR, DOW Chemical Company) was added together with HPMC K100M to prepare a bilayer tablet for oral administration.

**[Table 9]**

| Polmacoxib-tramadol combination (Example 9) | | | | | | |
|---|---|---|---|---|---|---|
| Tramadol layer | | Manufacturing process | Purpose | Material | per tablet (mg) | Content (%) |
| | 1 | First granulation | Main ingredient | Tramadol | 75 | 37.5 |
| | 2 | | Excipient | MCC 102 | 15 | 7.5 |
| | 3 | | Sustained-release agent | HPMC K100M | 70 | 35 |
| | 4 | | Sustained-release agent | PEO WSR 301 | 30 | 15 |
| | 5 | | Binder | PVP K-30 | 7.5 | 3.8 |
| | 6 | Post-mixing | Lubricant | Magnesium stearate | 2.5 | 1.3 |
| | Total amount of uncoated tablet | | | | 200 | 100 |
| Polmacoxib layer | 7 | First granulation | Main ingredient | Polmacoxib | 1 | 2 |
| | 8 | | Disintegrant | Sodium starch glycolate | 3.5 | 7 |
| | 9 | | Excipient | Silicified microcrystalline cellulose | 43.5 | 87 |
| | 10 | | Binder | Povidone K-30 | 1.5 | 3 |
| | 11 | Post-mixing | Lubricant | Magnesium stearate | 0.5 | 1 |
| | Total amount of uncoated tablet | | | | 50 | 100 |

### Example 10

In consideration of tableting properties, PEO was removed and the HPMC content was reduced to prepare a bilayer tablet for oral administration.

**[Table 10]**

| Polmacoxib-tramadol combination (Example 10) | | | | | | |
|---|---|---|---|---|---|---|
| Tramadol layer | | Manufacturing process | Purpose | Material | per tablet (mg) | Content (%) |
| | 1 | First granulation | Main ingredient | Tramadol | 75 | 42.9 |
| | 2 | | Excipient | MCC 102 | 18.5 | 10.6 |
| | 3 | | Sustained-release agent | HPMC K100M | 70 | 40 |
| | 4 | | Binder | PVP K-30 | 7.5 | 4.3 |
| | 5 | Post-mixing | Excipient | Aerosil | 1.5 | 0.8 |
| | 6 | | Lubricant | Magnesium stearate | 2.5 | 1.4 |
| | Total amount of uncoated tablet | | | | 175 | 100 |
| Polmacoxib layer | 7 | First granulation | Main ingredient | Polmacoxib | 1 | 2 |
| | 8 | | Disintegrant | Sodium starch glycolate | 3.5 | 7 |
| | 9 | | Excipient | Silicified microcrystalline cellulose | 43.5 | 87 |
| | 10 | | Binder | Povidone K-30 | 1.5 | 3 |
| | 11 | Post-mixing | Lubricant | Magnesium stearate | 0.5 | 1 |
| | Total amount of uncoated tablet | | | | 50 | 100 |

### Example 11

Since the mass of the polmacoxib layer is lower than that of the tramadol layer, the content unevenness between the layers is occurred. In order to prevent this phenomenon, the amount of the excipient in the polmacoxib layer was increased by two times to prepare a bilayer tablet for oral administration.

**[Table 11]**

| Polmacoxib-tramadol combination (Example 11) | | | | | | |
|---|---|---|---|---|---|---|
| Tramadol layer | | Manufacturing process | Purpose | Material | per tablet (mg) | Content (%) |
| | 1 | First granulation | Main ingredient | Tramadol | 75 | 42.9 |
| | 2 | | Excipient | MCC 102 | 18.5 | 10.6 |
| | 3 | | Sustained-release agent | HPMC K100M | 70 | 40 |
| | 4 | | Binder | PVP K-30 | 7.5 | 4.3 |
| | 5 | Post-mixing | Excipient | Aerosil | 1.5 | 0.8 |
| | 6 | | Lubricant | Magnesium stearate | 2.5 | 1.4 |
| | Total amount of uncoated tablet | | | | 175 | 100 |
| Polmacoxib layer | 7 | First granulation | Main ingredient | Polmacoxib | 1 | 1 |
| | 8 | | Disintegrant | Sodium starch glycolate | 7 | 7.1 |
| | 9 | | Excipient | Silicified microcrystalline cellulose | 87 | 87.9 |
| | 10 | | Binder | Povidone K-30 | 3 | 3 |
| | 11 | | Solubilizer | Purified water | 40 | 40.4 |
| | 12 | Post-mixing | Lubricant | Magnesium stearate | 1 | 1 |
| | Total amount of uncoated tablet | | | | 99 | 100 |

### Examples 12 to 14

After the preparation of the formulation in the same manner as in Example 11, in order to confirm the pattern of release change according to the hardness of the bilayer tablet for oral administration, the formulation was prepared such that the tablets had hardness of 7, 10 and 13 kp, respectively.

### Example 15

A tablet composition containing 150 mg of tramadol and 2 mg of polmacoxib was prepared for development of tablets intended for twice a day (bis in die, BID) administration and at the same time for development of tablets intended for four times a day (quaque die, QD) administration.

In case that the total weight of the polmacoxib rapid-release layer in the BID tablet is 99 mg, as in Example 11 to form a thin layer, there is a possibility of occurrence of problems such as mass deviation and content unevenness during manufacturing. Therefore, the amount of the rapid-release layer was increased by two times to prepare a bilayer tablet for oral administration.

**[Table 12]**

| Polmacoxib-tramadol combination (Example 15) | | | | | | |
|---|---|---|---|---|---|---|
| Tramadol layer | | Manufacturing process | Purpose | Material | per tablet (mg) | Content (%) |
| | 1 | First granulation | Main ingredient | Tramadol | 150 | 31.9 |
| | 2 | | Excipient | MCC 101 | 20 | 4.2 |
| | 3 | | Excipient | Mannitol 160C | 20 | 4.2 |
| | 4 | | Sustained-release agent | HPMC K100M | 200 | 42.5 |
| | 5 | | Binder | PVP K-30 | 15 | 3.2 |
| | 6 | Post-mixing | Sustained-release agent | Carbomer 71G | 60 | 12.7 |
| | 7 | | Lubricant | Magnesium stearate | 5 | 1.1 |
| | Total amount of uncoated tablet | | | | 470 | 100 |
| Polmacoxib layer | 7 | First granulation | Main ingredient | Polmacoxib | 2 | 1 |
| | 8 | | Disintegrant | Sodium starch glycolate | 14 | 7.1 |
| | 9 | | Excipient | Silicified microcrystalline cellulose | 174 | 87.9 |
| | 10 | | Binder | Povidone K-30 | 6 | 3 |
| | 11 | | Solubilizer | Purified water | 80 | 40.4 |
| | 12 | Post-mixing | Lubricant | Magnesium stearate | 2 | 1 |
| | Total amount of uncoated tablet | | | | 198 | 100 |

### Example 16

The dissolution rate in Example 15 was higher than that of a reference drug, and thus Carbomer 71G was changed to Carbomer 974P as a sustained-release agent in post-mixing to prepare a composition.

Since Carbomer 974P has lower flowability of the particles than that of Carbomer 71G but exhibits high sustained release property, Carbomer 974P was added in an appropriate amount in consideration of flowability to prepare a composition.

**[Table 13]**

| Polmacoxib-tramadol combination (Example 16) | | | | | | |
|---|---|---|---|---|---|---|
| Tramadol layer | | Manufacturing process | Purpose | Material | per tablet (mg) | Content (%) |
| | 1 | First granulation | Main ingredient | Tramadol | 150 | 31.9 |
| | 2 | | Excipient | MCC 101 | 20 | 4.2 |
| | 3 | | Excipient | Mannitol 160C | 20 | 4.2 |
| | 4 | | Sustained-release agent | HPMC K100M | 200 | 42.5 |
| | 5 | | Binder | PVP K-30 | 15 | 3.2 |
| | 6 | Post-mixing | Sustained-release agent | Carbomer 974P | 90 | 12.7 |
| | 7 | | Lubricant | Magnesium stearate | 5 | 1.1 |
| | Total amount of uncoated tablet | | | | 500 | 100 |
| Polmacoxib layer | 7 | First granulation | Main ingredient | Polmacoxib | 2 | 1 |
| | 8 | | Disintegrant | Sodium starch glycolate | 14 | 7.1 |
| | 9 | | Excipient | Silicified microcrystalline cellulose | 174 | 87.9 |
| | 10 | | Binder | Povidone K-30 | 6 | 3 |
| | 11 | | Solubilizer | Purified water | 80 | 40.4 |
| | 12 | Post-mixing | Lubricant | Magnesium stearate | 2 | 1 |
| | Total amount of uncoated tablet | | | | 198 | 100 |

### Example 17

Although the dissolution rate of Example 16 was close to that of a reference drug, the flowability of Carbomer was not good in the production of the composition and there were mass deviation and a difficulty in production. Accordingly, PEO, another sustained-release agent was added to prepare a composition.

**[Table 14]**

| Polmacoxib-tramadol combination (Example 17) | | | | | | |
|---|---|---|---|---|---|---|
| Tramadol layer | | Manufacturing process | Purpose | Material | per tablet (mg) | Content (%) |
| | 1 | First granulation | Main ingredient | Tramadol | 150 | 29.4 |
| | 2 | | Excipient | MCC 101 | 20 | 3.9 |
| | 3 | | Excipient | Mannitol 160C | 20 | 3.9 |
| | 4 | | Sustained-release agent | PEO WSR 301 | 300 | 58.8 |
| | 5 | | Binder | PVP K-30 | 15 | 2.9 |
| | 6 | Post-mixing | Lubricant | Magnesium stearate | 5 | 0.9 |
| | Total amount of uncoated tablet | | | | 510 | 100 |
| Polmacoxib layer | 7 | First granulation | Main ingredient | Polmacoxib | 2 | 1 |
| | 8 | | Disintegrant | Sodium starch glycolate | 14 | 7.1 |
| | 9 | | Excipient | Silicified microcrystalline cellulose | 174 | 87.9 |
| | 10 | | Binder | Povidone K-30 | 6 | 3 |
| | 11 | | Solubilizer | Purified water | 80 | 40.4 |
| | 12 | Post-mixing | Lubricant | Magnesium stearate | 2 | 1 |
| | Total amount of uncoated tablet | | | | 198 | 100 |

### Examples 18 and 19

In order to reduce the high dissolution rate in Example 17, PEO was removed and high molecular weight of HPMC K200M and HPMC K100M CR were added to prepare a composition.

**[Table 15]**

| Polmacoxib-tramadol combination (Example 18) | | | | | | |
|---|---|---|---|---|---|---|
| Tramadol layer | | Manufacturing process | Purpose | Material | per tablet (mg) | Content (%) |
| | 1 | First granulation | Main ingredient | Tramadol | 150 | 29.4 |
| | 2 | | Excipient | MCC 101 | 20 | 3.9 |
| | 3 | | Excipient | Mannitol 160C | 20 | 3.9 |
| | 4 | | Sustained-release agent | HPMC K200M | 300 | 58.8 |
| | 5 | | Binder | PVP K-30 | 15 | 2.9 |
| | 6 | Post-mixing | Lubricant | Magnesium stearate | 5 | 0.9 |
| | Total amount of uncoated tablet | | | | 510 | 100 |
| Polmacoxib layer | 7 | First granulation | Main ingredient | Polmacoxib | 2 | 1 |
| | 8 | | Disintegrant | Sodium starch glycolate | 14 | 7.1 |
| | 9 | | Excipient | Silicified microcrystalline cellulose | 174 | 87.9 |
| | 10 | | Binder | Povidone K-30 | 6 | 3 |
| | 11 | | Solubilizer | Purified water | 80 | 40.4 |
| | 12 | Post-mixing | Lubricant | Magnesium stearate | 2 | 1 |
| | Total amount of uncoated tablet | | | | 198 | 100 |

**[Table 16]**

| Polmacoxib-tramadol combination (Example 19) | | | | | | |
|---|---|---|---|---|---|---|
| Tramadol layer | | Manufacturing process | Purpose | Material | per tablet (mg) | Content (%) |
| | 1 | First granulation | Main ingredient | Tramadol | 150 | 29.4 |
| | 2 | | Excipient | MCC 101 | 20 | 3.9 |
| | 3 | | Excipient | Mannitol 160C | 20 | 3.9 |
| | 4 | | Sustained-release agent | HPMC K100M CR | 300 | 58.8 |
| | 5 | | Binder | PVP K-30 | 15 | 2.9 |
| | 6 | Post-mixing | Lubricant | Magnesium stearate | 5 | 0.9 |
| | Total amount of uncoated tablet | | | | 510 | 100 |
| Polmacoxib layer | 7 | First granulation | Main ingredient | Polmacoxib | 2 | 1 |
| | 8 | | Disintegrant | Sodium starch glycolate | 14 | 7.1 |
| | 9 | | Excipient | Silicified microcrystalline cellulose | 174 | 87.9 |
| | 10 | | Binder | Povidone K-30 | 6 | 3 |
| | 11 | | Solubilizer | Purified water | 80 | 40.4 |
| | 12 | Post-mixing | Lubricant | Magnesium stearate | 2 | 1 |
| | Total amount of uncoated tablet | | | | 198 | 100 |

### Example 20

In order to reduce the dissolution rate, hardened vegetable oil and HPMC K200M were added to prepare a composition.

**[Table 17]**

| Polmacoxib-tramadol combination (Example 20) | | | | | | |
|---|---|---|---|---|---|---|
| Tramadol layer | | Manufacturing process | Purpose | Material | per tablet (mg) | Content (%) |
| | 1 | First granulation | Main ingredient | Tramadol | 150 | 29.4 |
| | 2 | | Excipient | MCC 101 | 20 | 3.9 |
| | 3 | | Excipient | Mannitol 160C | 20 | 3.9 |
| | 4 | | Sustained-release agent | HPMC K100M CR | 150 | 29.4 |
| | 5 | | Sustained-release agent | Hardened vegetable oil | 150 | 29.4 |
| | 6 | | Binder | PVP K-30 | 15 | 2.9 |
| | 7 | Post-mixing | Lubricant | Magnesium stearate | 5 | 0.9 |
| | Total amount of uncoated tablet | | | | 510 | 100 |
| Polmacoxib layer | 7 | First granulation | Main ingredient | Polmacoxib | 2 | 1 |
| | 8 | | Disintegrant | Sodium starch glycolate | 14 | 7.1 |
| | 9 | | Excipient | Silicified microcrystalline cellulose | 174 | 87.9 |
| | 10 | | Binder | Povidone K-30 | 6 | 3 |
| | 11 | | Solubilizer | Purified water | 80 | 40.4 |
| | 12 | Post-mixing | Lubricant | Magnesium stearate | 2 | 1 |
| | Total amount of uncoated tablet | | | | 198 | 100 |

### Examples 21 and 22

Based on the dissolution results of the above examples, Carbomer which shows dissolution rate closest to a reference drug was selected as a sustained-release agent and Carbomer was added to both of granulation and post-mixing to prepare a composition. An appropriate amount of Carbomer was added in post-mixing in consideration of flowability to prepare a composition. With the same composition, a tablet was prepared in the form of a circular punch in Example 21 and a tablet was prepared in the form of a rectangular punch in Example 22 in order to compare differences in dissolution rate according to the shape of tablet.

**[Table 18]**

| Polmacoxib-tramadol combination (Examples 21 and 22) | | | | | | |
|---|---|---|---|---|---|---|
| Tramadol layer | | Manufacturing process | Purpose | Material | per tablet (mg) | Content (%) |
| | 1 | First granulation | Main ingredient | Tramadol | 150 | 28.9 |
| | 2 | | Excipient | HPMC K100M CR | 160 | 30.8 |
| | 3 | | Excipient | Carbomer 974P | 150 | 28.9 |
| | 4 | | Binder | PVP K-30 | 15 | 2.9 |
| | 5 | Post-mixing | Sustained-release agent | Carbomer 974P | 40 | 7.7 |
| | 6 | | Lubricant | Magnesium stearate | 4 | 0.7 |
| | Total amount of uncoated tablet | | | | 519 | 100 |
| Polmacoxib layer | 7 | First granulation | Main ingredient | Polmacoxib | 2 | 1 |
| | 8 | | Disintegrant | Sodium starch glycolate | 14 | 7.1 |
| | 9 | | Excipient | Silicified microcrystalline cellulose | 174 | 87.9 |
| | 10 | | Binder | Povidone K-30 | 6 | 3 |
| | 11 | | Solubilizer | Purified water | 80 | 40.4 |
| | 12 | Post-mixing | Lubricant | Magnesium stearate | 2 | 1 |
| | Total amount of uncoated tablet | | | | 198 | 100 |

### Experimental Example 1: Evaluation of dissolution

Evaluation of dissolution was performed according to the dissolution test method of Table 19 below for evaluation of dissolution property of the formulation according to Example.

As a control group, ULTRACET sustained-release tablet (Janssen Korea, Ltd., ultracet ER tablet, ultracet ER Tab. Acetaminophen 650mg, Tramadol hydrochloride 75mg) was used in Comparative Example 1, ACELEX Tab. (Crystalgenomics, Acelex tablet, 2mg-Polmacoxib) was used in Comparative example 2 and Zytram XL (Mundipharma Korea, Zytram XL sustained-release tablet, Tramadol hydrochloride 150mg) was used in Comparative Example 3.

**[Table 19]**

| | Test method 1 (Tramadol) | Test method 2 (Polmacoxib) |
|---|---|---|
| Test method | Test 2 (Paddle method) of dissolution test of Korean Pharmacopoeia | Test 2 (Paddle method) of dissolution test of Korean Pharmacopoeia |
| Test solution | Solution 1 of dissolution test of Korean Pharmacopoeia, 900 mL | Water, 900 mL |
| Test temperature | 37 ± 0.5 °C | 37 ± 0.5 °C |
| Rotation speed | 50 rpm | 50 rpm |

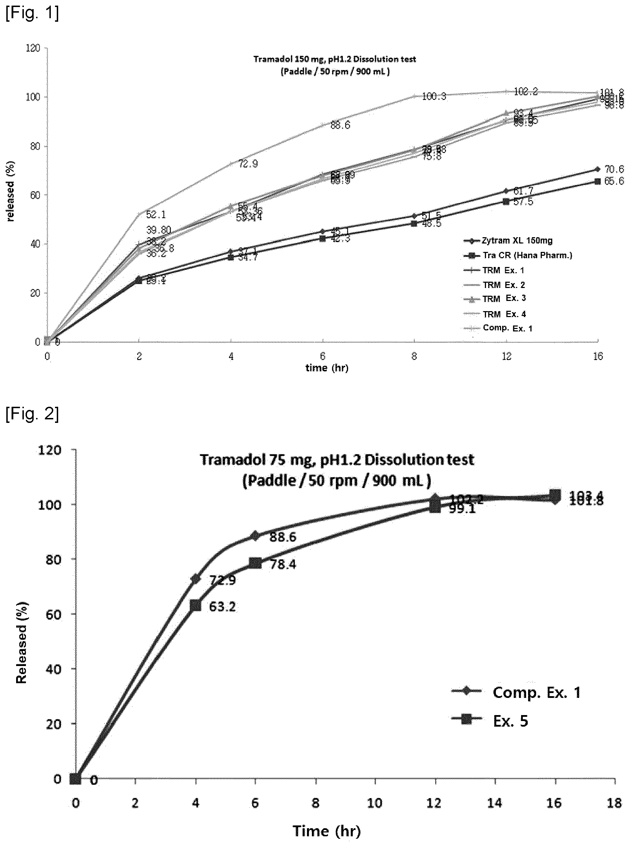

The dissolution evaluation results of Examples 1 to 4 are shown in Table 20 and Fig. 1, wherein the unit of dissolution rate is %.

**[Table 20]**

| | | | | | | | | |
|---|---|---|---|---|---|---|---|---|
| pH 1.2 | | 0hr | 2hr | 4hr | 6hr | 8hr | 12hr | 16hr |
| | Ultracet ER 75mg | 0 | 52.1 | 72.9 | 88.6 | 100.3 | 102.2 | 101.8 |
| | Zytram XL 75mg | 0 | 31.2 | 43.0 | 51.6 | 58.3 | 68.2 | 76.4 |
| | Zytram XL 150mg | 0 | 26.2 | 37.1 | 45.1 | 51.5 | 61.7 | 70.6 |
| | Tra CR (HANA Pharm) | 0 | 25.1 | 34.7 | 42.3 | 48.5 | 57.5 | 65.6 |
| | TRM Example 1 | 0 | 39.80 | 53.36 | 68.39 | 78.58 | 90.65 | 99.15 |
| | TRM Example 2 | 0 | 36.2 | 53.4 | 65.9 | 75.8 | 89.5 | 96.8 |
| | TRM Example 3 | 0 | 38.2 | 55.4 | 67.9 | 78.4 | 93.4 | 100.2 |
| | TRM Example 4 | 0 | 36.8 | 53.4 | 66.7 | 77.3 | 91.0 | 98.0 |

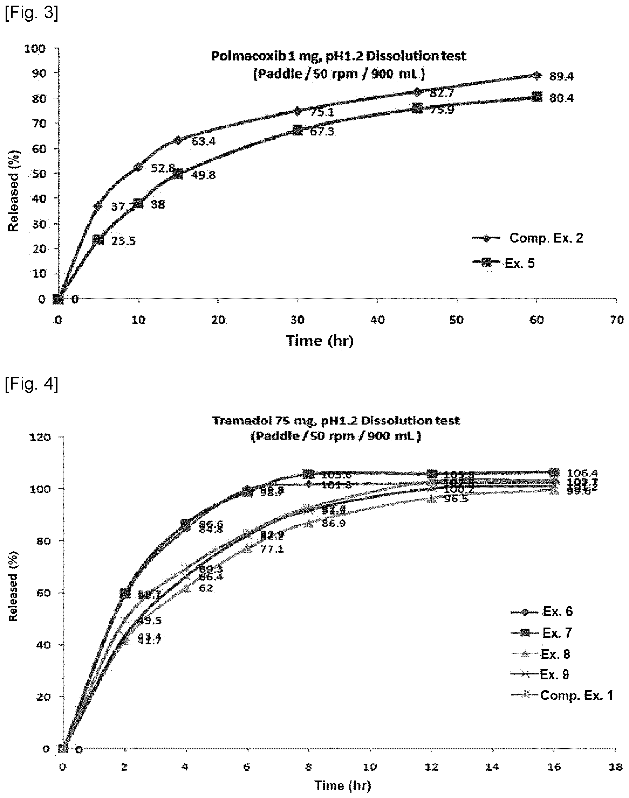

The dissolution evaluation results of tramadol layer in Example 5 are shown in Table 21 and Fig. 2 and the dissolution evaluation results of polmacoxib layer in Example 5 are shown in Table 22 and Fig. 3, wherein the unit of dissolution rate is %.

**[Table 21]**

| | 0hr | 4hr | 6hr | 12hr | 16hr |
|---|---|---|---|---|---|
| Comparative Example 1 | 0 | 72.9 | 88.6 | 102.2 | 101.8 |
| Example 5 | 0 | 63.2 | 78.4 | 99.1 | 103.4 |

**[Table 22]**

| | 0hr | 5hr | 10hr | 15hr | 30hr | 45hr | 60hr |
|---|---|---|---|---|---|---|---|
| Comparative Example 2 | 0 | 37.2 | 52.8 | 63.4 | 75.1 | 82.7 | 89.4 |
| Example 5 | 0 | 23.5 | 38 | 49.8 | 67.3 | 75.9 | 80.4 |

The dissolution evaluation results of Examples 6 to 9 are shown in Table 23 and Fig. 4, wherein the unit of dissolution rate is %.

**[Table 23]**

| | 0hr | 2hr | 4hr | 6hr | 8hr | 12hr | 16hr |
|---|---|---|---|---|---|---|---|
| Example 6 | 0 | 59.1 | 84.8 | 99.8 | 101.8 | 102.3 | 102.7 |
| Example 7 | 0 | 59.7 | 86.6 | 98.7 | 105.6 | 105.8 | 106.4 |
| Example 8 | 0 | 41.7 | 62 | 77.1 | 86.9 | 96.5 | 99.6 |
| Example 9 | 0 | 43.4 | 66.4 | 82.2 | 91.9 | 100.2 | 101.2 |
| Comparative Example 1 | 0 | 49.5 | 69.3 | 82.9 | 92.7 | 102.8 | 103.1 |

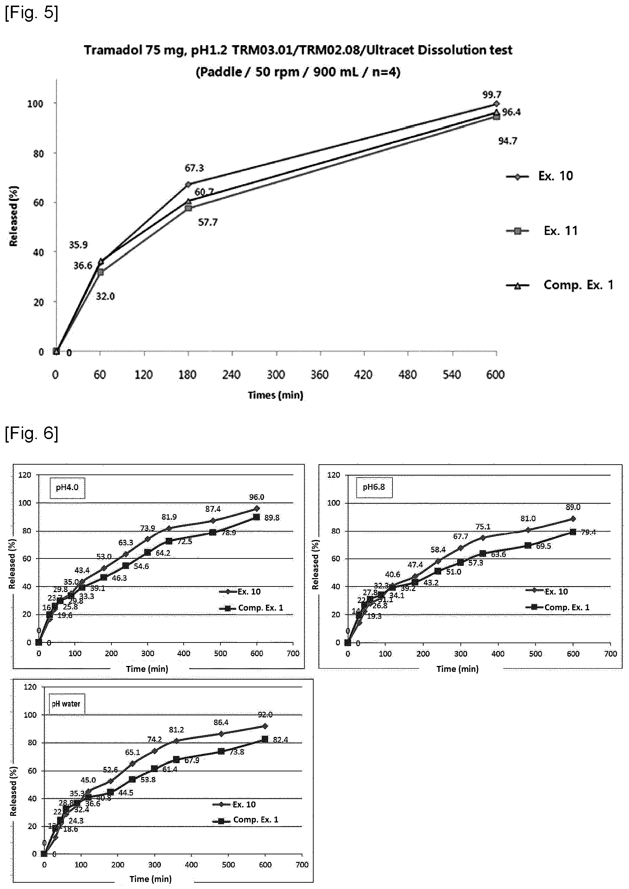

The dissolution evaluation results of Examples 10 and 11 are shown in Table 24 and Fig. 5. As shown in Fig. 5, it was confirmed that Example 10 exhibits a dissolution pattern similar to that of Comparative Example 1. In addition, the dissolution evaluation results according to pH of Example 10 are shown in Table 25 and Fig. 6. The unit of dissolution rate is%.

**[Table 24]**

| | min | 0 | 60 | 180 | 600 |
|---|---|---|---|---|---|
| Example 11 | Average | 0 | 35.9 | 67.3 | 99.7 |
| | Standard deviation | 0 | 0.8 | 0.8 | 1.5 |
| Example 10 | Average | 0 | 32.0 | 57.7 | 94.7 |
| | Standard deviation | 0 | 1.8 | 2.2 | 3.4 |
| Comparative Example 1 | Average | 0 | 36.6 | 60.7 | 96.4 |
| | Standard deviation | 0 | 0.3 | 0.8 | 0.6 |

**[Table 25]**

| | | | | | | | | | | | | | |
|---|---|---|---|---|---|---|---|---|---|---|---|---|---|
| pH water | | 0 | 30 | 45 | 60 | 90 | 120 | 180 | 240 | 300 | 360 | 480 | 600 |
| | Example 10 | 0 | 12.1 | 22.2 | 28.8 | 35.3 | 45.0 | 52.6 | 65.1 | 74.2 | 81.2 | 86.4 | 92.0 |
| | Comparative Example 1 | 0 | 18.6 | 24.3 | 32.4 | 36.6 | 40.8 | 44.5 | 53.8 | 61.4 | 67.9 | 73.8 | 82.4 |
| pH 6.8 | | 0 | 30 | 45 | 60 | 90 | 120 | 180 | 240 | 300 | 360 | 480 | 600 |
| | Example 10 | 0 | 14.2 | 22.2 | 27.8 | 32.3 | 40.6 | 47.4 | 58.4 | 67.7 | 75.1 | 81.0 | 89.0 |
| | Comparative Example 1 | 0 | 19.3 | 26.8 | 31.1 | 34.1 | 39.2 | 43.2 | 51.0 | 57.3 | 63.6 | 69.5 | 79.4 |
| pH 4.0 | | 0 | 30 | 45 | 60 | 90 | 120 | 180 | 240 | 300 | 360 | 480 | 600 |
| | Example 10 | 0 | 16.5 | 23.7 | 29.8 | 35.0 | 43.4 | 53.0 | 63.3 | 73.9 | 81.9 | 87.4 | 96.0 |
| | Comparative Example 1 | 0 | 19.6 | 25.8 | 29.8 | 33.3 | 39.1 | 46.3 | 54.6 | 64.2 | 72.5 | 78.9 | 89.8 |

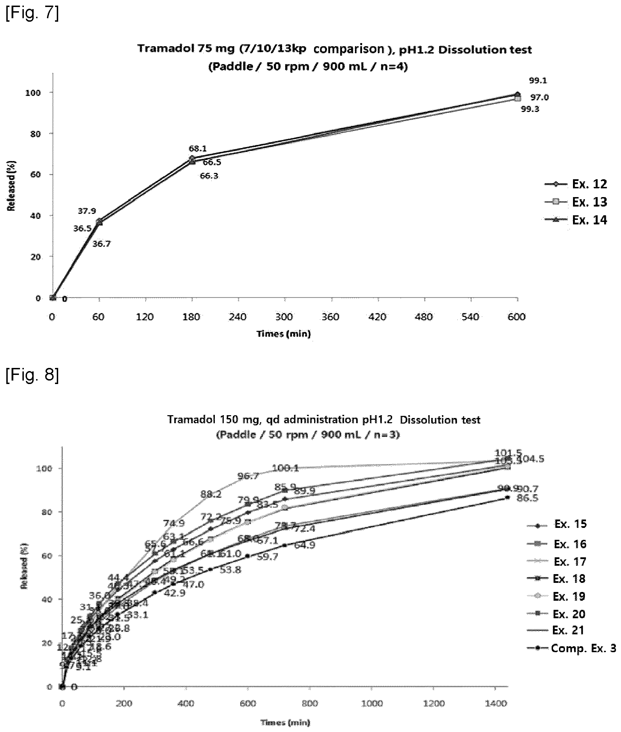

The dissolution evaluation results of Examples 12 to 14 are shown in Table 26 and Fig. 7, wherein the unit of dissolution rate is %.

**[Table 26]**

| | min | 0 | 60 | 180 | 600 |
|---|---|---|---|---|---|
| Example 12 (hardness 7kp) | Average | 0 | 37.9 | 68.1 | 99.1 |
| | Standard deviation | 0 | 0.8 | 0.5 | 0.6 |
| Example 13 (hardness 10kp) | Average | 0 | 36.5 | 66.5 | 97.0 |
| | Standard deviation | 0 | 0.6 | 0.4 | 0.9 |
| Example 14 (hardness 13kp) | Average | 0 | 36.7 | 66.3 | 99.3 |
| | Standard deviation | 0 | 0.4 | 0.3 | 0.4 |

The dissolution evaluation results of Examples 15 to 21 and Comparative Example 3 are shown in Table 27 and Fig. 8, wherein the unit of dissolution rate is %.

**[Table 27]**

| | | | | | | | | | | | | | | |
|---|---|---|---|---|---|---|---|---|---|---|---|---|---|---|
| pH1.2 | | 0 | 15 | 30 | 60 | 90 | 120 | 180 | 300 | 360 | 480 | 600 | 720 | 1440 |
| | Example 15 | 0 | 12.5 | 17.7 | 25.1 | 31.1 | 36.0 | 44.4 | 57.7 | 63.1 | 72.2 | 79.9 | 85.9 | 101.5 |
| | Example 16 | 0 | 9.7 | 14.1 | 20.5 | 25.5 | 29.7 | 37.0 | 48.4 | 53.1 | 61.1 | 68.0 | 73.7 | 90.9 |
| | Example 17 | 0 | 9.7 | 14.5 | 22.5 | 29.0 | 35.0 | 46.3 | 65.6 | 74.9 | 88.2 | 96.7 | 100.1 | 103.5 |
| | Example 18 | 0 | 9.4 | 14.0 | 21.0 | 27.1 | 31.9 | 40.2 | 52.9 | 58.6 | 67.9 | 75.5 | 81.6 | 100.4 |
| | Example 19 | 0 | 8.9 | 13.4 | 19.9 | 25.4 | 30.3 | 39.5 | 52.5 | 57.9 | 67.6 | 75.3 | 82.1 | 101.0 |
| | Example 20 | 0 | 12.2 | 17.6 | 26.0 | 32.3 | 38.3 | 47.2 | 61.1 | 66.6 | 75.9 | 83.5 | 89.9 | 104.5 |
| | Example 21 | 0 | 11.1 | 15.5 | 21.9 | 27.2 | 31.5 | 38.4 | 49.2 | 53.5 | 61.0 | 67.1 | 72.4 | 90.7 |
| | Comparative Example 3 | 0 | 9.1 | 12.8 | 18.6 | 23.0 | 26.8 | 33.1 | 42.9 | 47.0 | 53.8 | 59.7 | 64.9 | 86.5 |

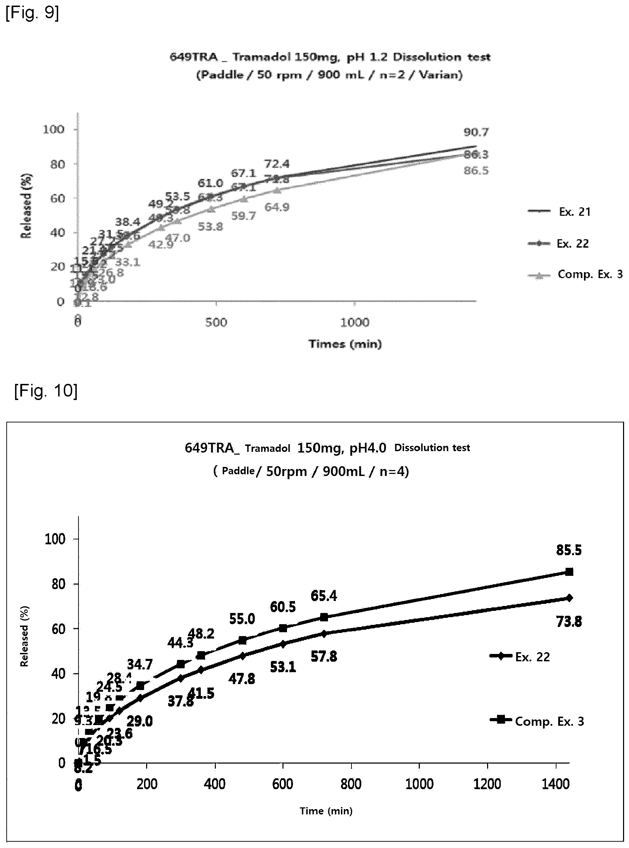

The dissolution evaluation results of Example 22 are shown in Table 28 and Fig. 9, wherein the unit of dissolution rate is %.

**[Table 28]**

| | | | | | | | | | | | | | | |
|---|---|---|---|---|---|---|---|---|---|---|---|---|---|---|
| pH1.2 | | 0 | 15 | 30 | 60 | 90 | 120 | 180 | 300 | 360 | 480 | 600 | 720 | 1440 |
| | Example 22 | 0 | 10.9 | 15.5 | 22.2 | 27.2 | 31.5 | 38.6 | 49.3 | 53.8 | 61.3 | 67.1 | 71.8 | 86.3 |
| | Comparative Example 3 | 0 | 9.1 | 12.8 | 18.6 | 23.0 | 26.8 | 33.1 | 42.9 | 47.0 | 53.8 | 59.7 | 64.9 | 86.5 |

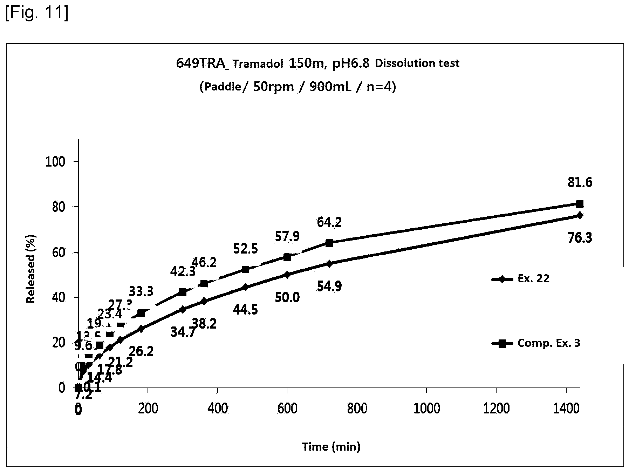

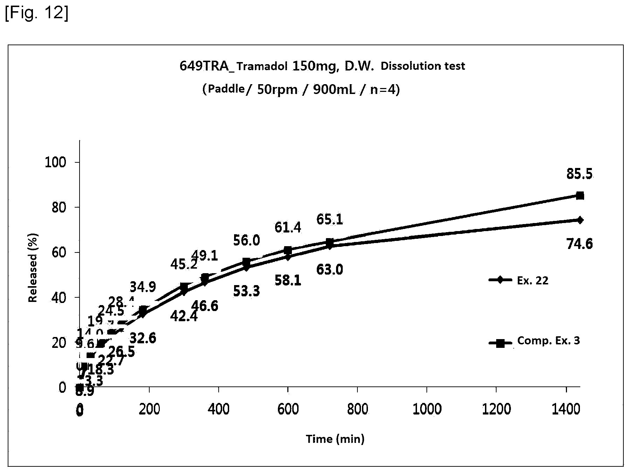

The dissolution evaluation results at pH 6.8 and pH 4.0 and pH water of Example 22 and Comparative Example 3 are shown in Table 29 and Figs. 10 to 12, wherein the unit of dissolution rate is %.

**[Table 29]**

| | | | | | | | | | | | | | | |
|---|---|---|---|---|---|---|---|---|---|---|---|---|---|---|
| pH4.0 | | 0 | 15 | 30 | 60 | 90 | 120 | 180 | 300 | 360 | 480 | 600 | 720 | 1440 |
| | Example 22 | 0 | 8.2 | 11.5 | 16.5 | 20.3 | 23.6 | 29.0 | 37.8 | 41.5 | 47.8 | 53.1 | 57.8 | 73.8 |
| | Comparative Example 3 | 0 | 9.3 | 13.5 | 19.8 | 24.5 | 28.4 | 34.7 | 44.3 | 48.2 | 55.0 | 60.5 | 65.4 | 85.5 |
| pH6.8 | | 0 | 15 | 30 | 60 | 90 | 120 | 180 | 300 | 360 | 480 | 600 | 720 | 1440 |
| | Example 22 | 0 | 7.2 | 10.1 | 14.4 | 17.8 | 21.2 | 26.2 | 34.7 | 38.2 | 44.5 | 50.0 | 54.9 | 76.3 |
| | Comparative Example 3 | 0 | 9.6 | 13.5 | 19.1 | 23.4 | 27.3 | 33.3 | 42.3 | 46.2 | 52.5 | 57.9 | 64.2 | 81.6 |
| pH water | | 0 | 15 | 30 | 60 | 90 | 120 | 180 | 300 | 360 | 480 | 600 | 720 | 1440 |
| | Example 22 | 0 | 8.9 | 13.3 | 18.3 | 22.7 | 26.5 | 32.6 | 42.4 | 46.6 | 53.3 | 58.1 | 63.0 | 74.6 |
| | Comparative Example 3 | 0 | 9.6 | 14.0 | 19.9 | 24.5 | 28.4 | 34.9 | 45.2 | 49.1 | 56.0 | 61.4 | 65.1 | 85.5 |

### Experimental Example 2: Evaluation of therapeutic effect of osteoarthritis

In order to evaluate therapeutic effect of osteoarthritis, an animal model of osteoarthritis was prepared in Sprague Dawley rats (n = 10 per group).
1) Monosodium iodoacetate (MIA, I2512, Sigma, Poole, UK) was dissolved in 0.9% sterile saline for injection at a concentration of 60 mg/ml and prepared on the day of the experiment (day 0).
2) After group separation, animals (Sprague Dawley rats) were placed in an anesthesia chamber (Matrix, US / VME-2) and anesthetized using diethyl ether. Thereafter, subjects in the induced group (G2 to G9) were injected once with 50 µl of MIA per subject (MIA 3 mg/head) into a left knee joint space via a patellar tendon using an insulin syringe (BDTM insulin syringe with Ultra-Fine™ Short Needle, 31 gauge). The number of animals in each group was 10, and the negative control group (G1) was injected once with 50 µl of saline at the same position in the left knee joint.

Group separation was performed only on animals judged to have no quarantine abnormalities during acclimation period, followed by MIA administration. On the 6th day after MIA administration, the weight bearing of hind leg for a total of 100 subjects administered with MIA was measured and the affected subjects with high weight bearing of left hind leg were sequentially excluded to select 70 subjects. Selected subjects were randomly grouped to have uniform distribution of body weight load and body weight. After completion of the group separation, an individual identification card (including test name, test group, individual number, sex, beginning date of test, etc.) was attached to each breeding box. They were separated into a total of seven groups and the detailed description of each group is shown in Table 30 below.

**[Table 30]**

| Group | Detailed description |
|---|---|
| G1 | Normal group (Administration of saline, negative control group) |
| G2 | Induced group |
| G3 | Ultracet (Acetaminophen and tramadol hydrochloride co-administration, positive control group) |
| G4 | polmacoxib single administration |
| G5 | Tramadol hydrochloride single administration |
| G6 | polmacoxib and tramadol hydrochloride co-administration (low dose) |
| G7 | polmacoxib and tramadol hydrochloride co-administration (high dose) |

In Table 30, in the groups G6 and G7, the content ratio of polmacoxib and tramadol hydrochloride is set to 1: 100 and the dose in the group G7 is 1.5 times higher than the dose in the group G6. That is, in the group G6, 0.2 mg/kg of polmacoxib and 20 mg/kg of tramadol hydrochloride are administrated, and in the group G7, 0.3 mg/kg of polmacoxib and 30 mg/kg of tramadol hydrochloride are administrated. Specific administration information of each group is as follows:
G1: Normal group, no substances to be administered during the test period
G2: Induced group, no substances to be administered during the test period
G3: Acetaminophen 300mg/kg + Tramadol hydrochloride 30mg/kg
G4: Polmacoxib 0.3mg/kg
G5: Tramadol hydrochloride 30mg/kg
G6: Polmacoxib 0.2mg/kg + Tramadol hydrochloride 20mg/kg
G7: Polmacoxib 0.3mg/kg + Tramadol hydrochloride 30mg/kg.

Each group was orally administered with 3 mL once daily for 3 weeks.

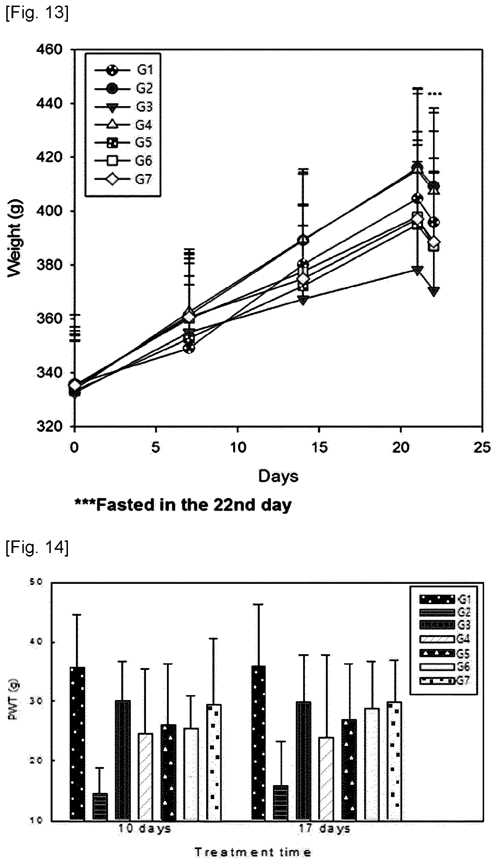

The average weight range of each group at the beginning of the test was from 332.58 ± 22.92g to 335.93 ± 25.62g and the measurement results of weight change are shown in Fig. 8. After 3 weeks, the average weight of each group at the end of the test was from 378.38 ± 40.03g to 415.26 ± 30.11g, specifically G1 (20.5%), G2 (24.5%), G3 (13.8%), G4 (24.4%), G5 (18.5%), G6 (18.6%) and G7 (18.4%) compared to the average weight of each group at the beginning of the test, which indicate a weight gain of about 13.8% to 24.5%. For the weight change for 3 weeks, the test groups showed no significant difference compared to the normal group G1. However, the G3 group showed the slowest weight gain and a significant weight loss on day 21 and before autopsy after fasting (Fig. 13). In all test groups, no clinical symptoms or deaths related to administration of the test substance were observed during the test period.

### Experimental Example 3: Evaluation of analgesic efficacy

To evaluate analgesic efficacy, paw withdrawal threshold (PWT, g) was measured using a dynamic planter aesthesiometer (Ugo Basile S.R.L, Italy).

Von Frey filament used in this experiment has a constant pressure during bending, thereby imparting a constant stimulation. At this time, physical allodynia appears as an avoidance response, thereby enabling quantification of the pain index.
1) The pain suppression effect was measured by imparting physical stimulation with Von Frey filament using Dynamic plantar aesthesiometer (UGO BASILE 37450, Italy).
2) Rats were placed individually in each acrylic cage, allowing the rats to stay for 15 minutes to habituate to the new environment.
3) After habituation, the aesthesiometer was set to 0 ∼ 50g and 0 ∼ 20s. The measurement of allodynia by stimulation of the stimulator is performed by stimulating the bottom part of the affected foot, increasing the force incrementally (0-50 g), and measuring the hind paw withdraw threshold (force at the moment when the test body avoided the stimulator: gram) in triplicate at 5 min intervals.
4) The paw withdrawal threshold (PWT) was measured in duplicate at a fixed time within 2 weeks (day 10) and within 3 weeks (day 17) after administration of the test substance.

The measurement results are shown in Fig. 14. As a result of observing the avoidance response after physically developing allodynia using Dynamic plantar aesthesiometer, it was found that the change of stimulus intensity of the avoidance response during the 3-week evaluation period was 32.1 ∼ 39.5g for the negative control group G1, whereas the change was 10.5 ∼ 18.9g for the induced group G2, which indicates that a dramatic decrease in a mechanical threshold is maintained (Fig. 14). In all test groups, the paw withdrawal threshold was found to be increased compared to the G2 group. From the 10-day evaluation, it was found that the paw withdrawal threshold was 30.3g, 24.2g, and 26.3g for the positive control group G3 (acetaminophen and tramadol co-administration), the group G4 (polmacoxib single administration) and the group G5 (tramadol hydrochloride single administration), respectively, and the paw withdrawal threshold was 25.5g and 29.59g for the combination of co-administration groups G6 and G7. That is, all treatment groups showed a significant increase in the paw withdrawal threshold, compared to the induced group G2 which shows 14.7g of PWT (p<0.05). As a result of evaluating the paw withdrawal threshold (PWT), the positive control group G3, the tramadol hydrochloride single administration group G5, the co-administration groups G6 and G7 showed 30.0 g, 26.3 g, 26.2 g, and 31.8 g, respectively, and the polmacoxib single administration group G4 showed 24.2g, which indicates a significant increase compared to the induced group G2 (p <0.05).

In this evaluation, it was found that the paw withdrawal threshold tends to be more increased in the high-dose of polmacoxib and tramadol hydrochloride co-administration G7 than in the low-dose group G6, with regard to the analgesic efficacy against mechanical allodynia. The average paw withdrawal threshold in the test groups was similar between the positive control group G3 and the high-dose of polmacoxib and tramadol hydrochloride co-administration G7, which is expected to show similar analgesic effects. The group G6 (polmacoxib 0.2mg/kg + tramadol 20mg/kg) showed a similar pain relief effect to that of using tramadol (30mg/kg) alone, despite the reduced amount of tramadol by 1/3. It is a result that can expect reduction of the risk of side effects and addiction that may occur with long-term use of tramadol.

### Experimental Example 4: Evaluation of anti-inflammatory effect

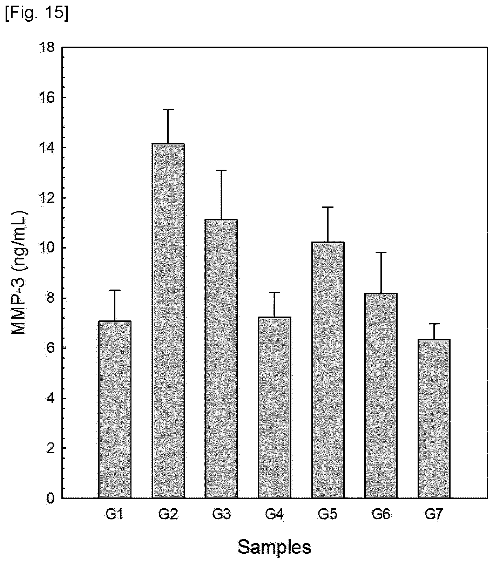

Matrix metalloproteinase (MMP) is responsible for the controlled degradation of cellular substrates in normal physiological environments. It has been reported to be a factor that causes tissue damage by extensive degradation of cellular substrate components in case that its action is uncontrolled and overexpressed. In the present study, the level of MMP-3 active ingredients in serum obtained after 3 weeks of treatment was lower in all treatment groups compared to the induced group (G2). In comparison of treatment groups, the polmacoxib group and the polmacoxib + tramadol combination group showed lower MMP-3 levels compared to the groups G3 and G5. From this, it can be expected that polmacoxib which is a potent anti-inflammatory drug can inhibit activity of catabolic factor (MMP-3) and thus inhibit joint damage, thereby producing a synergistic effect on pain relief (Fig. 15).

In conclusion, the combination of polmacoxib and tramadol can be used as an excellent pain treatment that has excellent pain relief effect and anti-inflammatory effect and has a very low expression of side effect, compared with the combination of acetaminophen + tramadol or tramadol alone as a reference drug.

### Experimental Example 5: Histological evaluation

Tissue sections were stained for histological evaluation.
1) Tissues of liver and stomach among isolated organs were sufficiently immobilized in 10% neutral buffered formalin solution, followed by general tissue treatment procedures such as trimming, dehydration and paraffin embedding to prepare tissue sections and cut them to about 4 µm. Thereafter, they were stained with Hematoxylin & Eosin (H&E) and examined microscopically.
2) The collected knee joint tissues were immobilized in 10% formalin for 24 hours, and decalcificated for 72 hours while exchanging the solution with 10% formic acid at a 24-hour time interval prior to tissue treatment. The sagittal section of the knee joint was prepared through a coronal section so that the medial and lateral condyle articular surface of femur and tibia were well identified.
3) Subsequently, after a general tissue treatment procedure such as dehydration, clearing and penetration, paraffin embedding was performed and a 5 µm thin slice of knee joint was prepared with a rotary microtome. It was subject to deparaffinization and hydration, followed by Hematoxylin & Eosin (H&E) staining.
4) For safranin-O fast green (SOFG) staining, after deparaffinization and hydration, the thin slice of knee joint was reacted in Weigert's iron hematoxylin solution (HT110232, Sigma, St Louis, USA) for 10 minutes and washed with water, and then stained in 0.02% fast green(FCF) solution (F7258, Sigma, St Louis, USA) for 5 minutes. Subsequently, it was reacted in 1% acetic acid solution and then stained with 0.1% safranin O solution (S2255, Sigma, St Louis). The slide stained with safranin O-fast green was washed for 5 minutes to have clear color of nucleus. After dehydration and clarification, it was sealed.
5) In order to observe destruction of cartilage and bone, damage of articular cartilage and histopathologic changes and evaluate the progression of degenerative arthritis, the following Mankin score (max = 19) was used to classify and digitize the degree of surface damage of articular cartilage and the degree of safranin O staining.
   Criteria for histopathological scoring of osteoarthritis lesions
   - Modified-Mankin scoring system
   Variable score (Max 19)
   Surface
      0 = Intact surface
      1 = Surface fissures
      2 = Surface fissures to midzone
      3 = Surface fissures to deep zone
      4 = Complete disorganization
   Hypocellularity
      0 = Inta
      1 = slightdecrease in chondrocytes
      2 = moderatedecrease in chondrocytes
      3 = severe decrease in chondrocytes
      4 = no cells
   Clone/Osteophyte formations
      0 = normal
      1 = occasional duos
      2 = duos or trios
      3 = multiple nested cells
   Stain intensity for safranin o
      0 = normal
      1 = slight reduction
      2 = moderate reduction
      3 = severe reduction
      4 = no dye noted
   Structure
      0= normal
      1 = erosion in < 15% surface
      2 = erosion in < 50% surface
      3 = ulceration in < 75% surface
      4 = ulceration in ≥ 75% surface

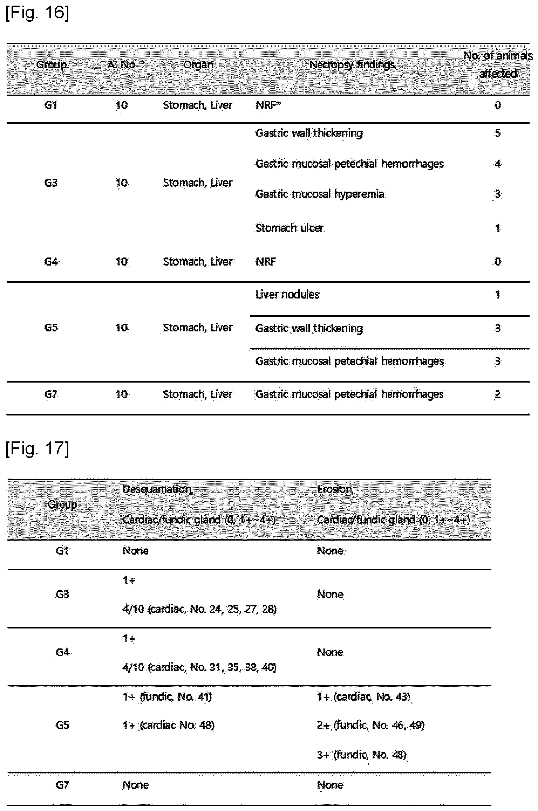

   Mankin scores are the sum of each index.
6) In order to confirm the effect of each sample on the site other than the treatment site, gross observation was performed after the necropsy. The results are shown in Figs. 15 and 16.

As shown in Fig. 15, as a result of gross necropsy observation after completion of the experiment, no unusual abnormal findings were identified in the negative control group G1 and the polmacoxib single administration group G4 and 5 cases of gastric wall thickening, 4 cases of gastric mucosal petechial hemorrhage, 3 cases of gastric mucosal hyperemia and 1 case of stomach ulcer were identified in the positive control group G3 showing significant weight loss, which was identified as the group with the most incidence of abnormalities among the treatment groups. 1 case of nodules on the surface of liver, 3 cases of gastric wall thickening and 3 cases of gastric mucosal petechial hemorrhage were identified in the tramadol single administration group G5. 2 cases of gastric mucosal petechial hemorrhage were identified in the high-dose of polmacoxib and tramadol hydrochloride co-administration group G7.

As shown in Fig. 16, from tissue pathology findings, 4 cases of mild level of epithelial detachment of cardiac gland were observed in the positive control group G3, 4 cases of mild level of epithelial detachment of cardiac gland similar to the positive control group G3 were observed in the polmacoxib single administration group G4, and 2 cases of epithelial detachment in cardiac gland as well as 4 cases of erosion of cardiac gland and fundic gland were identified in the tramadol hydrochloride single administration group G5. No abnormal findings were observed in the high-dose of polmacoxib and tramadol co-administration group G7 as in the negative control group G1. As a result of histological examination of the liver, in the negative control group G1, the hepatocytes had normal lobular architecture maintained around the central vein while the radial hepatic cell cords and sinusoids showed cord-like arrangement toward the edge of the hepatic lobules. All test groups showed similar findings to the negative control group G1, indicating that there was no specific abnormality associated with administration of the test substance.

In conclusion, the co-administration of polmacoxib and tramadol hydrochloride, a test substance, alleviated pain in the MIA-induced osteoarthritis rat model, compared to single administration of each substance. It leads to the increased use of affected hind leg. Therefore, there were synergistic effect of improving weight distribution ratio and alleviating perceptual hypersensitivity of mechanical allodynia. In general, it was found that similar or better analgesic effects were exhibited in the polmacoxib and tramadol hydrochloride co-administration groups G6 and G7 compared to the positive control group G3 (acetaminophen and tramadol hydrochloride co-administration). In addition, in the histological evaluation, the degrees of damage of joint surface, chondrocyte reduction, and glycosaminoglycan reduction were similar in all the test groups. However, with regard to cluster formation of chondrocytes, which is another indicator of damage, the tramadol hydrochloride single administration group G5 and the polmacoxib and tramadol hydrochloride co-administration groups G6 and G7 showed improvement effects compared to the positive control group G3 (acetaminophen and tramadol co-administration). As a result of gastric tissue analysis, epithelial detachment of pyloric was observed in the polmacoxib single administration group G4 and epithelial detachments of cardia and pyloric and erosion were observed in the tramadol single administration group G5, which indicate gastric irritation. In the monosodium iodoacetate (MIA) osteoarthritis rat model under these test conditions, it was confirmed that the polmacoxib and tramadol hydrochloride co-administration groups G6 and G7 showed equivalent analgesic efficacy without gastric irritation compared to the positive control group G3 (acetoaminophen and tramadol hydrochloride co-administration).

The above description is merely illustrative of the technical idea of the present invention, and various modifications and variations can be made by those skilled in the art without departing from the essential characteristics of the present invention. In addition, the embodiments disclosed in the present invention are not intended to limit the technical idea of the present invention but to describe the present invention, and the scope of the technical idea of the present invention is not limited by these embodiments. The protection scope of the present invention should be interpreted by the following claims, and all technical ideas within the scope equivalent thereto should be construed as being included in the scope of the present invention.

## Claims

1. A pharmaceutical composition for treating acute or chronic pain, comprising polmacoxib and tramadol.

2. The pharmaceutical composition according to claim 1, wherein the pain is neurological pain, including diabetic neuropathy, cancer pain, osteoarthritis, moderate pain, rheumatoid arthritis, spondylitis or sciatica.

3. The pharmaceutical composition according to claim 1, wherein the composition is in the form of a double-layered tablet having a bi-layered structure in which the polmacoxib and the tramadol are separated into each layer.

4. The pharmaceutical composition according to claim 1, wherein the composition further comprises a coating base.

5. The pharmaceutical composition according to claim 1, wherein the composition is in the form of a cored tablet formulation containing an inner core of tramadol and an outer core of polmacoxib or a dual release micro-coating (DRM) containing an outer coating layer of polmacoxib on an inner layer of tramadol.

6. The pharmaceutical composition according to claim 1, wherein the ratio of polmacoxib to tramadol is a weight ratio of 1: 1 to 500.

7. The pharmaceutical composition according to claim 6, wherein the ratio of polmacoxib to tramadol is a weight ratio of 1: 1 to 300.

8. The pharmaceutical composition according to claim 6, wherein the ratio of polmacoxib to tramadol is a weight ratio of 2: 1 to 300.

9. The pharmaceutical composition according to claim 1, wherein the composition further comprises a pharmaceutically acceptable carrier.

10. The pharmaceutical composition according to claim 1, wherein the composition is in the form of tablet, pellet, granule, capsule, suspension, emulsion or powder.
